# EUROPÄISCHE PATENTSCHRIFT

(11) **EP 2 670 705 B1**
(45) Veröffentlichungstag und Bekanntmachung des Hinweises auf die Patenterteilung: **01.07.2020**
(21) Anmeldenummer: 12703741.4
(22) Anmeldetag: 31.01.2012
(51) Int. Cl.: A61L 26/00, A61L 27/48, C01B 33/145, C01B 33/148

(54) **KIESELSÄUREKONDENSATE MIT GERINGER VERNETZUNG IN EINER POLYMERMATRIX**
SILICIC ACID CONDENSATES HAVING A LOW DEGREE OF CROSS-LINKING IN A POLYMER MATRIX
CONDENSATS D'ACIDE SILICIQUE À FAIBLE RÉTICULATION INCORPORÉS DANS UN MATRICE POLYMÈRE

(30) Priorität: 31.01.2011 DE 102011009838; 31.01.2011 DE 102011009839
(43) Veröffentlichungstag der Anmeldung: 11.12.2013
(73) Patentinhaber: Gerber, Thomas, 18059 Sildemow (DE)
(72) Erfinder: Gerber, Thomas, 18059 Sildemow (DE)
(74) Vertreter: Uexküll & Stolberg
(86) Internationale Anmeldenummer: PCT/EP2012/051581
(87) Internationale Veröffentlichungsnummer: WO 2012/104310

(56) Entgegenhaltungen:
- WO-A1-2006/128793
- WO-A2-01/52618
- WO-A2-2005/087284
- WO-A2-2009/030919
- DATABASE WPI Week 201058 Thomson Scientific, London, GB; AN 2010-L26448 XP002677862, & CN 101 786 139 A (DONGGUAN WELL SILICASOL CO LTD) 28. Juli 2010 (2010-07-28)

## Beschreibung

Gegenstand der Erfindung ist ein Material oder Biomaterial, umfassend Kieselsäurekondensate mit geringer Vernetzung, und Verfahren zu dessen Herstellung. Es wird ein Verfahren zur Herstellung gering vernetzter Kieselsäurestrukturen offenbart, bei dem man ein Sol erzeugt, wobei man eine weitere Kondensation verhindert, wenn bestimmte Vernetzungen der Kieselsäure erreicht werden, wobei bevorzugt Strukturen einer Größe von 0,5-20 nm hergestellt werden, z.B. Polyederstrukturen oder Aggregate derselben. Eine weitere Kondensation kann durch Mischung mit einem Polyvinylpyrrolidon-Polymer verhindert werden. In einer Ausführungsform umfasst das nanostrukturiertes, gering vernetztes Siliziumdioxid (SiO₂), das in einer Polyvinylpyrrolidon-Polymermatrix eingebettet ist. Das Material kann in der Medizin für therapeutische Zwecke eingesetzt werden und dabei einen unmittelbaren Kontakt mit biologischem Gewebe des Körpers eingehen. Dieses Material tritt dabei in chemische, physikalische und biologische Wechselwirkungen mit den entsprechenden biologischen Systemen. Es kann dabei abgebaut werden und als Lieferant für die im Stoffwechsel benötigte Kieselsäure wirken. Es kann darüber hinaus eine stützende oder abschirmende Wirkung haben. Es kann als Granulat, Mikropartikel, Faser und daraus hergestelltes Gewebe oder Vlies oder als Schicht auf Implantaten oder Wundverbänden vorliegen. Das Material kann als Medizinprodukt oder als Nahrungsergänzung verwendet werden.

Seit den 70iger Jahren des letzten Jahrhunderts ist bekannt, dass Silizium ein wichtiges Spurenelement für den Aufbau von Knochen und Kollagen ist. (siehe z.B. M. Carlisle; Silicon: An Essential Element for the Chick; Science 10 November 1972:Vol. 178. no. 4061, pp. 619 - 621). Die genauen biochemischen Prozesse sind nach wie vor nicht bekannt. Im Stoffwechsel kommt Silizium in erster Linie als Silizumdioxid vor. Auch ist nicht bekannt, in welcher Struktur das Siliziumdioxid am besten am Stoffwechsel teilnimmt. Siliziumdioxid kommt als kristalline Verbindung (z.B. Quarz, Cristobalit), als Glas und als amorphe Substanz vor. Im Kristall und im Glas ist das Siliziumdioxid durch eine nahezu vollständige Vernetzung der SiO_{4/2} Tetraeder bestimmt. Das amorphe Siliziumdioxid mit dem wesentlichen Vertreter Kieselgel hat dagegen ein Netzwerk, das nicht kontinuierlich ist und durch mehr oder weniger innere Oberfläche mit offenen Bindungen (meist SiOH) gekennzeichnet ist.

In Bezug auf eine Degradation von Siliziumdioxid ist die Löslichkeit von Siliziumdioxid in Wasser im Bereich des physiologischen pH-Wertes interessant. Sie liegt für amorphes SiO₂ bei pH 7 bei ca. 150 ppm. Im Kontakt mit lebendem Gewebe erfolgt eine schnellere Lösung als in Pufferlösung pH 7,4. Der Grund hierfür ist unbekannt (Iler, The Chemistry of Silica, 1979 John Wiley &Sons).

Für Wundauflagen wird im Patent US005741509A eine Mischung von Silikonmedium mit "fumed silica" beschrieben. "Fumed silica" besteht aus nichtporösen SiO₂ Partikeln mit einer Dichte von 2,2 g/cm³ und einer Größe zwischen 5 und 50 nm (Wikipedia). Die Dichte, die identisch mit der des Kieselglases ist, zeigt ebenso wie die fehlende Porosität, dass es sich um vollständig vernetzte SiO₂ Strukturen handelt.

Im Patent US2004/0235574A1 wird ebenfall eine Mischung von Silikonmedium mit "fumed silica" beschrieben, wobei zusätzlich antibakteriell wirkende Substanzen hinzugegeben werden.

Das Patent US 7,074,981 B2 beschreibt eine Wundauflage, in der ein Absorbens oder ein Adsorbens in Form von Kieselgel verwendet wird. Ein Absorbens oder ein Adsorbens aus Kieselgel ist nach Stand der Technik ein Xerogel, das im Allgemeinen aus Natriumwasserglaslösung hergestellt wird, wobei eine für ein Xerogel typische Vernetzung der SiO₂ Strukturen erfolgt. (Unter Wikipedia "Adsorption": "Silica gel is a chemically inert, nontoxic, polar and dimensionally stable (< 400 °C or 750 °F) amorphous form of SiO₂. It is prepared by the reaction between sodium silicate and acetic acid, which is followed by a series of after-treatment processes such as aging, pickling, etc. These after treatment methods results in various pore size distributions".)

Mit biologisch degradierbaren Fasern unter anderem aus SiO₂, deren Herstellung und deren Verwendung als Verstärkungsfasern, beschäftigt sich das Patent DE 196 09 551 C1. Hier wird die Herstellung eines spinnbaren Sols beschrieben. Das beschriebene Verfahren und die beschriebenen Anwendung basieren auf der Diplomarbeit von Monika Kursawe von 1995. Die Diplomarbeit basiert wiederum auf den ursprünglichen Synthesevorschriften von Sakka von 1982 (S. Sakka, K. Kamiya; J.Non-Cryst.Solids 48, 1982 31). Sakka beschreibt ein Verfahren, bei dem Gelfäden gesponnen werden, aus denen dann in einem späteren Schritt Glasfasern hergestellt werden. Ausgangsmaterial ist Tetraethylorthosilikat (TEOS), wobei durch Hydrolyse und Kondensation ein spinnfähiges Sol erzeugt wird. Schon Sakka zeigt, dass durch die thixotropen Eigenschaften der Sole nur ein begrenzter Bereich in der Zusammensetzung (TEOS, H₂O, Lösungsmittel (im Allgemeinen Ethanol) und Katalysator) zu spinnbaren Solen führt. Insbesondere das Molverhältnis von Wasser zu TEOS muss um r_{w} 2 liegen.

In der Dissertation "Entwicklung eines Verfahrens zur Herstellung degradierbarer Kieselgelfasern für die Medizintechnik" (Monika Kursawe 1999), einer Weiterentwicklung der Diplomarbeit von 1995 von Kursawe, steht, dass der wichtigste Unterschied zwischen dem in ihrer Diplomarbeit beschriebenen und dem Verfahren von Sakka der ist, dass nach der Kondensation eine Solreifung eingeführt wurde und das als Katalysator Salpetersäure anstatt Salzsäure verwendet wurde. In der Dissertation wird dann das Verfahren so optimiert, dass die Herstellung hochwertiger Kieselgelfasern in größeren Mengen erfolgen kann. Der Prozess basiert auf der leicht modifizierten, in der Diplomarbeit beschriebenen Synthesevorschrift von Sakka.

Das Patent DE 37 80 954 T2 beschreibt ein Verfahren von Siliziumdioxid-Glasfasern, wobei auch hier das Grundverfahren von Sakka modifiziert wurde.

Das Patent DE 10 2007 061 873 A1 beschreibt die Herstellung eines Kieselsolmaterials und die Verwendung als biologisch resorbierbares Material. Als Stand der Technik wird das Patent DE 19609551C1 herangezogen. Als Abgrenzung zu diesem Patent wird aufgeführt, dass hier die Fasern nach dem Spinnen keine optimalen Ergebnisse bei Zytotoxizitätstests erreichen, wobei die Ursachen hierfür vielfältig sein können und nicht mit den wesentlichen Verfahrensschritten zusammenhängen. Auch die zweite Abgrenzung, dass es nach DE 10 2007 061 873 A1 zur Bildung einer "festen Phase" kommt, die ein Filtern des Sols erzwingt, wird nicht mit den wesentlichen Verfahrensschritten in Bezug gesetzt. Der Hauptanspruch 1 des Patentes DE 10 2007 061 873 A1 gibt im Wesentlichen die Herstellanweisung wieder, die M. Kusawe in ihrer Dissertation (1999) beschreibt und die auch in ihrer Diplomarbeit 1995 veröffentlich ist. M. Kusawe teilt die Herstellung des spinnbaren Sols in "Hydrolyse", "Kondensation" und "Reifung" ein. Die "Kondensation" ist nach Kusawe dadurch geprägt, dass dem Sol Ethanol entzogen wird. Das entspricht Anspruch 1 b) des Patentes DE 10 2007 061 873 A1. Die "Reifung" bei Kusawe erfolgt bei ihrem Standardansatz bei 5°C. Das wiederum entspricht dem Anspruch 1 c) und 1 d). Im Beispiel des Patentes DE 10 2007 061 873 A1 wird die Reifung bei 4°C durchgeführt. Auch in den anderen wesentlichen Parametern entspricht das Beispiel dem Standardansatz von Kusawe (z.B. Molverhältnis Wasser/TEOS 1.75, bei Kusawe 1.8). Die Verfahren zur Herstellung spinnbarer Sole der Patente DE 196 09 551 C1 und DE 10 2007 061 873 A1 gehen in ihren wesentlichen Schritten nicht über den Kenntnisstand der Diplomarbeit von Kusawe von 1995 hinaus. Auch die Diplomarbeit basiert stark auf dem Kenntnisstand von Sakka von 1982 (S. Sakka, K.Kamiya; J.Non-Cryst. Solids 48, 1982 31).

CN 101 786 139 A offenbart ein Verfahren zur Herstellung eines Kieselsäure-Sols, das 20-36% (w/w) SiO₂ und 0.1-0.5% (w/w) Polyvinylpyrrolidon enthält. WO 2009/030919 A2 offenbart ein Biomaterial, das in einem Sol durch Hydrolyse von Silanen hergestellt wird und aus SiO₂ Polyederstrukturen besteht, wobei kein Polyvinylpyrrolidon verwendet wird.

Aufgabe der vorliegenden Erfindung ist es, die Struktur von Kieselsäurekondensationsprodukten so zu optimieren, dass eine gesteuerte Degradation bei *in vivo* Anwendung erfolgen kann, und dass diese Kieselsäure-Kondensationsprodukte in bestimmten Applikationsformen wie Granulat, Mikroteilchen, Fasern oder als Schichten auf Implantaten oder Wundauflagen vorliegen können. Hierzu sollen auch Herstellungsverfahren bereitgestellt werden.

Erfindungsgemäß wird diese Aufgabe dadurch gelöst, dass die Kondensation der Kieselsäure in wässriger bzw. in alkoholischer Lösung so gesteuert wird, dass definierte Polyederstrukturen entstehen und dass diese Polyederstrukturen bei den folgenden Verfahrensschritten wie z.B. dem Entfernen des Lösungsmittels erhalten bleiben. Ziel ist, gering vernetzte Kieselsäurestrukturen zu erzeugen, die dadurch gekennzeichnet sind, das sie nicht in einem kontinuierlichen Netzwerk, wie es das Kieselglasnetzwerk ist, eingebaut sind. Der geringste Vernetzungsgrad stellt dabei ein Polyeder aus SiO₂ Tetraedern dar, wobei Fünfer-, Sechs- bzw. Siebenerringe eine räumliche Struktur von ca. 0,5 nm Durchmesser bilden. Solche Strukturen sind beschrieben in: B. Himmel, Th. Gerber and H. Bürger: WAXS- and SAXS-investigations of structure formation in alcoholic SiO2 solutions, Journal of Non-Crystalline Solids, Amsterdam, 119(1990)1-13; B. Himmel, Th. Gerber and H. Bürger: X-ray diffraction investigations of silica gel structures, Journal of Non-Crystalline Solids, Amsterdam, 91(1987)122-136; B. Himmel, Th. Gerber, W. Heyer and W. Blau: X-ray diffraction analysis of SiO2 structure, Journal of Material Science, Chapman and Hall Ltd., London, 22(1987)1374-1378; Th. Gerber and B. Himmel: The structure of silica glass in dependence on the fictive temperature, Journal of Non-Crystalline Solids, Amsterdam, 92(1987)407-417; Th. Gerber and B. Himmel: The structure of silica glass, Journal of Non-Crystalline Solids, Amsterdam, 83(1986)324-334; B. Himmel, Th. Gerber and H.-G. Neumann: X-ray diffraction investigations of differently prepared amorphous silicas, Physica Status Solidi (a), 88(1985) K127-K130).

Ein Ausgangsmaterial für die Herstellung von Kieselsäurekondensationprodukten ist Tetraethylorthosilikat (TEOS). Mit Wasser entsteht bei Anwesenheit von einem Katalysator Kieselsäure, wobei das Molverhältnis von Wasser/TEOS mindestens 4 sein muss, um zum Startpunkt eine vollständige Hydrolyse zu erreichen. Die entstandene Monokieselsäure kondensiert und bildet Polyederstrukturen von ca. 0.5 nm - 1 nm heraus, sogenannte Primärteilchen, die dann in einer Cluster-Cluster Aggregation fraktale Cluster herausbilden. Diese Cluster wachsen durch den Aggregationsprozess und bei einer bestimmten Größe der Cluster kommt es zur Gelbildung. D.h., die Cluster füllen durch ihre Packung bzw. durch das entstandene Perculationsnetzwerk das Gefäß aus (Th. Gerber, B. Himmel and C. Hübert: WAXS and SAXS investigation of structure formation of gels from sodium water glass, Journal of Non-Crystalline Solids, (1994) Bd. 175, S. 160-168 und B. Knoblich, Th. Gerber. C.F. Brinker und G.W. Scherer beschreiben die Gelbildung in einem extra Kapitel in "Sol-gel-science: the physikcs and chemistry of sol-gel processing" (Academic Press; San Diego; 1990). Die Gelbildung ist durch eine extreme Erhöhung der Viskosität charakterisiert.

Auf der Basis von Natriumwasserglaslösung können diese oder analoge Strukturen erzeugt werden. Hierbei werden die Natriumionen bevorzugt mit einem Ionenaustauscher aus der Lösung entfernt. Die verbleibende Kieselsäure liegt hier dann schon als Kondensationsprodukt vor. Es handelt sich um Polyederstrukturen von ca. 0,5 nm Größe, wiederum Primärteilchen genannt, die dann in Anhängigkeit vom pH-Wert durch Aggregation fraktale Cluster bilden, die wiederum zur Gelbildung führen (B. Knoblich, Th. Gerber: Aggregation in SiO2 sols from sodium silicate solutions, Journal of Non-Crystalline Solids 283 (2001) 109-113).

Die Aggregationscluster (Feststoffgerüst, Metalloxid) des Alkogels (Lösungsmittel, Alkohol) bzw. des Hydrogels (Lösungsmittel, Wasser) werden beim Trocknen aufgrund der wirkenden Kapillarkräfte und der ablaufenden Kondensation der inneren Oberfläche (2Si*_{surface}*-OH → Si*_{bulk}*-O-M*_{bulk}* + H₂O) zerstört. Es entsteht ein Xerogel, dessen innere Oberfläche z.B. in dem Fall SiO₂ im Bereich von 25-700 m²/g und dessen Dichte im Bereich über 1,0 g/cm³ liegt. Die definierten Polyederstrukturen in den Primärteilchen vernetzen sich beim Trocknen. Es entsteht ein kontinuierliches Netzwerk mit der oben beschriebenen hohen inneren Oberfläche. Die Vernetzung des Siliziumdioxides wird erhöht.

Bei der Herstellung von Aerogelen wird dieser Prozess verhindert. Hierfür gibt es nach dem Stand der Technik zwei grundlegend verschiedene Wege.

Zum einen werden überkritische Trocknungsverfahren angewandt. Hierdurch wird die Wirkung der Kapillarkräfte verhindert, da der Phasenübergang Flüssig/Gas durch ein entsprechendes Temperatur-Druck-Regime umgangen wird. Als Lösungsmittel werden hierbei Alkohole (Methanol, Ethanol, Propanol) oder flüssiges CO₂ verwendet, die durch Austauschverfahren das ursprüngliche Lösungsmittel, meist H₂O, ersetzen müssen (S.S. Kistler, Phys. Chem. 36 (1932) 52-64, EP 171722; DE 1811353; US 3672833; DE 39 24 244 A1; PCT/EP94/02822). Durch die verwendeten Autoklaven sind die Verfahren sehr kostenaufwändig.

Andererseits existieren nach dem Stand der Technik Verfahren, die eine unterkritische Trocknung von Aerogelen erlauben. Kernpunkt des Verfahrens nach PCT/US94/05105 ist eine Modifikation des Kontaktwinkels zwischen Lösungsmittel und Feststoffgerüst. Hierdurch wird der Kapillardruck verringert und die Struktur des feuchten Gels nahezu erhalten. Der Kontaktwinkel wird durch eine Modifikation der inneren Oberfläche des Feststoffgerüstes im feuchten Gel erreicht. Hierzu erfolgt eine Reaktion der inneren Oberfläche mit RₓSiX_{y}. R ist eine organische Gruppe und X ist ein Halogen. Bei diesem Verfahren ist ein mehrfacher Lösungsmittelaustausch notwendig. Im Patent DE 19538333 A1 wird eine Modifikation der inneren Oberfläche des feuchten Gels mit Si*_{surface}*O-Z realisiert, wobei Z eine beliebige Gruppe ist, die die Kondensation der inneren Oberfläche beim Trocknen verhindern soll.

Die zur Herstellung von Aerogelen verwendeten Verfahren werden im Rahmen der vorliegenden Erfindung nicht genutzt, insbesondere kommt es nicht zu einer Gelbildung.

Die Aufgabe der vorliegenden Erfindung ist es, Materialien mit einer definierten Vernetzung der Kieselsäure herzustellen. Daraus können Produkte wie Mikroteilchen, Fasern oder Schichten hergestellt werden.

Erfindungsgemäß wird diese Aufgabe dadurch gelöst, dass eine weitere Kondensation in einem Sol verhindert wird, wenn bestimmte Vernetzungen der Kieselsäure erreicht werden, insbesondere wenn die gewünschte Größe der Kieselgelcluster bzw. der Solteilchen durch Kondensation erreicht wurde, wobei die gewünschte Größe der Kieselgelcluster bzw. der Solteilchen bevorzugt im Bereich von ca. 0,5 nm bis hin zu ca. 1000 nm, mehr bevorzugt von 0,5 nm bis 20 nm, mehr bevorzugt von 0,5 nm bis 10 nm, von 0,5 nm bis 5 nm, von 0,5 nm bis 4 nm, von 0,5 nm bis 3 nm, von 0,5 nm bis 2 nm oder von 0,5 nm bis 1 nm ist.

Das geschieht dadurch, dass eine Lösung, insbesondere eine wässrige Lösung, eines löslichen Polyvinylpyrrolidon (PVP)-Polymers hinzu gegeben wird. Ein besonders bevorzugtes PVP ist K90. Es wird bevorzugt gemischt, bis die Kieselsäurestrukturen homogen in dem Polymer verteilt sind. Insbesondere kann eine Homogenisierung z.B. mit einem Ultraschallhomogenisator vorgenommen werden. Ein Mischen mit einem Rührer mit hohen Scherkräften hat sich ebenso als effektiv erwiesen. In einer Ausführungsform wird nach dem Mischen der pH-Wert auf ca. 6-8, insbesondere ca. pH 7 bis ca. pH 7,4 eingestellt wird.

Überraschenderweise kommt es dabei zu keiner Ausfällung der Kieselsäure. Es entsteht ein Gel, in dem die SiO₂ Kondensationsprodukte und die Moleküle des Polyvinylpyrrolidon-Polymers, homogen verteilt sind.

Dieses Gel kann z.B. Anwendung finden in Salben oder Cremes für die Wundbehandlung, für die Behandlung von Narben oder für kosmetische Anwendungen.

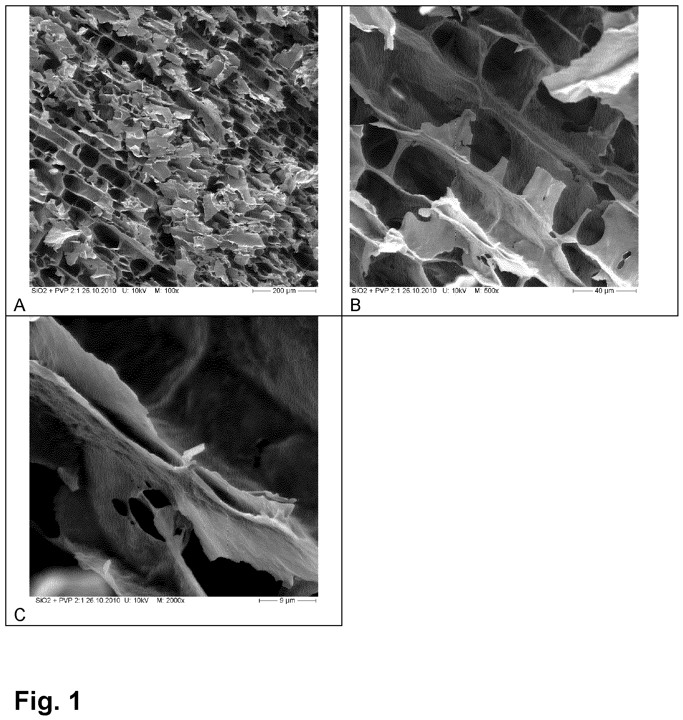

Beim Trocknen, bevorzugt beim Gefriertrocknen der Mischung, bleiben Polymer und Kieselsäurepolyeder homogen verteilt (siehe Fig. 1). Bei einer Nutzung als Wundauflage kann das Polymer wiederum in Lösung gehen und die Kieselsäurepolyeder so freisetzen.

Aus einem Polyvinylpyrrolidon-Polymer/SiO₂/Lösungsmittelgemisch können auch spinnfähige Lösungen hergestellt werden, wobei das Lösungsmittel bevorzugt Wasser ist. Hierzu wird bevorzugt das Verhältnis von Polyvinylpyrrolidon-Polymer, SiO₂ und Lösungsmittel so gewählt, dass nach dem Mischen die Viskosität im Bereich von ca. 0,7 - 1,3 Pas, insbesondere bei ca. 1 Pas liegt. Die Lösung kann sofort durch Düsen gedrückt werden. In einem Spinnturm können die Fäden im temperierten Gasstrom trocknen.

Gegenstand der Erfindung ist in dieser Ausführungsform insbesondere ein Verfahren, um gering vernetzte Kieselsäurestrukturen in einer Polymermatrix zu erzeugen, wobei:
a) ein SiO₂ Sol in einem Lösungsmittel erzeugt wird, wobei die Solteilchen bevorzugt eine Größe von 0,5 nm bis 20 nm haben,
b) eine Lösung eines Polymers in einem Lösungsmittel hergestellt wird,
c) die Lösung und das Sol homogen vermischt werden,
wobei das Lösungsmittel Wasser oder Alkohol oder ein Gemisch aus Wasser und Alkohol ist, wobei der Alkohol bevorzugt Ethanol ist, und wobei das Polymer Polyvinylpyrrolidon (PVP) ist, und wobei SiO₂ und Polymer in Schritt c) in einem Massenverhältnis (SiO₂/Polymer) von 0,5/99,5 bis 50/50 vorliegen.

In einer Ausführungsform der Erfindung wird der pH-Wert des Sols in Schritt a) im Bereich von 6 bis 8 eingestellt. In Schritt b) kann ein pH-Wert der Polymerlösung im Bereich von 6 bis 8 eingestellt werden.

In einer anderen Ausführungsform der Erfindung wird das Sol hergestellt, ohne den pH einzustellen. Der pH des Sols liegt dann bevorzugt bei ca. pH 2. Auch die Polymerlösung für Schritt b) kann hergestellt werden, ohne den pH einzustellen, wobei der pH dieser Lösung dann, z.B. bei PVP bevorzugt bei ca. 4 liegt. In dieser Ausführungsform wird der pH bevorzugt nach dem Mischen in Schritt c) auf 6-8 eingestellt, insbesondere auf pH 7.

Das Mischen wird insbesondere durchgeführt, bis eine homogene Verteilung der Kieselsäurestrukturen in dem Polymer erreicht ist. Möglich ist z.B. ein Mischen für ca. 6 min bei Rühren von 1000 rpm.

Bei dem erfindungsgemäßen Verfahren liegen SiO₂ und PVP im Massenverhältnis 0,5/99,5 (SiO₂/Polymer) bis 50/50 vor (in Schritt c) bzw. im Endprodukt).

Bevorzugt ist bei dem erfindungsgemäßen Verfahren das Polymer Polyvinylpyrrolidon K90.

Bevorzugt wird bei diesem Verfahren das Gemisch aus Schritt c) getrocknet. Das Gemisch kann in eine Form gegossen und gefriergetrocknet werden.

Bevorzugt wird soviel Lösungsmittel aus dem Gemisch entfernt, das die Viskosität bevorzugt im Bereich von 0,5 Pas bis 1 Pas liegt. Dadurch können Fäden gesponnen werden, die bevorzugt in einem temperierten Gasstrom getrocknet werden. Aus den Fäden kann ein Vlies oder ein Gewebe hergestellt werden.

Im Rahmen dieses Verfahrens kann der Vernetzungsgrad des Polymers, z.B. des PVP, nach bekannten Methoden, bevorzugt durch Gammabestrahlung, erhöht werden.

Gegenstand der Erfindung ist auch ein Material, welches mit einem erfindungsgemäßen Verfahren herstellbar ist.

Gegenstand der Erfindung ist auch ein Material, welches als Biomaterial bezeichnet werden kann, und welches aus SiO₂ Strukturen, insbesondere Polyederstrukturen aufgebaut ist, die eine Größe von bevorzugt 0,5 nm bis 4 nm aufweisen, und die in einer Polyvinylpyrrolidon-Polymermatrix homogen verteilt sind. Bevorzugt ist das Material ein Hydrogel, aufgebaut aus SiO₂ Strukturen, die bevorzugt eine Größe von 0,5 nm bis 4 nm aufweisen, und Polyvinylpyrrolidon-umfasst, wobei Polyvinylpyrrolidon-und SiO₂ Strukturen homogen verteilt sind. Die gering vernetzten Kieselsäurestrukturen sind insbesondere Polyederstrukturen oder Aggregate derselben. Aggregate von Polyederstrukturen sind aus Polyederstrukturen aufgebaut.

In diesem Material liegen SiO₂ und Polyvinylpyrrolidon-im Massenverhältnis 0,5/99,5 (SiO₂/Polymer) bis 50/50 vor.

Bevorzugt ist in diesem Material das Polymer Polyvinylpyrrolidon K90.

Bevorzugt liegt dieses Material als Fäden, die bevorzugt ein Vlies oder Gewebe bilden, als Folie oder als Schwamm vor.

In einer Ausführungsform bildet dieses Material (Biomaterial) zusammen mit als Knochenersatzmaterial nutzbaren oder genutzten Granulaten eine Masse zum Füllen von Knochendefekten (Putty). Es können handelsübliche Granulate verwendet werden.

Solche Granulate sind z.B. in WO 2004/103421 und EP 1 624 904 offenbart. Es kann z.B. Nanobone (Artoss GmbH, Rostock, Deutschland) verwendet werden. Besonders bevorzugt sind hoch poröse Knochenersatzmaterialgranulate, insbesondere Granulate auf Basis von Calciumphosphat, wie kristallines Calciumphosphat das in einer Siliziumdioxid-Xerogelmatrix eingebettet ist. Solche Granulate auf Basis von Calciumphosphat, sind insbesondere erhältlich durch Herstellung des Calciumphosphats über eine Fällungsreaktion, bei der man die Lösung mit dem ausgefällten Calciumphosphat durch Rühren homogenisiert, man eine hochkonzentrierte Kieselsäurelösung zugibt, man das Gemisch durch die anschließend einsetzende Gelbildung fixiert und man es durch Entfernen des Lösungsmittels in eine Xerogelmatrix überführt, wobei die in der Xerogelmatrix liegenden Calciumphosphatkristallite eine Größe von etwa 10 nm bis etwa 2000 nm und die Granulatkörner eine Größe von 1 µm bis 1000 µm aufweisen und der Siliziumdioxid-Anteil im Bereich von 2 bis 80 Gew.-%, vorzugsweise im Bereich von 4 bis 50 Gew.-%, bezogen auf die Gesamtmasse der Granulatkörner liegt. Ferner können auch Knochenersatzmaterialien in Granulatform bovinen Ursprungs (z.B. BioOss der Firma Geistlich) oder Hydroxylapatitkeramiken (z.B. gesinterte Hydroxylapatitkeramiken, wie z.B. Cerabone - aap Implantate AG) im Rahmen der vorliegenden Erfindung verwendet werden. Es ist allerdings zu berücksichtigen, dass bei einer Putty auf Wasserbasis keine wasserlöslichen Granulate (z.B. β-TCP) verwendet werden können.

Gegenstand der Erfindung ist auch eine Verwendung des Materials für Medizinprodukte, insbesondere für solche, die eine stützende oder abschirmende Funktion haben und/oder gleichzeitig durch die Degradation als Lieferant für das die Geweberegeneration unterstützende Siliziumdioxid dienen. Im Rahmen der Erfindung wird die Bezeichnung Medizinprodukt als austauschbar zu medizinische oder pharmazeutische Zusammensetzung oder Medikament verwendet, da die Klassifizierung von nationalem Recht abhängt, die Substanz der Erfindung jedoch nicht ändert.

Gegenstand der Erfindung ist auch eine Verwendung des Materials als Salbe oder Creme, insbesondere für die Behandlung von Wunden, Narben oder für kosmetische Anwendungen.

Gegenstand der Erfindung ist auch ein Medizinprodukt, welches das erfindungsgemäße Material umfasst.

Die vorliegende Erfindung betrifft ferner Verfahren zur Herstellung eines formbaren Knochenersatzmaterials (Putty), bei denen man a) ein als Knochenersatzmaterial nutzbares Granulat mit einer wässrigen Lösung anfeuchtet, und b) das als Knochenersatzmaterial nutzbare Granulat mit einem, wie oben beschrieben hergestellten Biomaterial vermischt. Granulate, die im Rahmen dieses Verfahren verwendet werden können, umfassen die oben offenbarten Granulate.

In einer besonderen Ausführungsform wird die in Schritt b) hergestellte Mischung gefriergetrocknet, so dass der Anwender (z.B. ein Chirurg) dem entstandenen Schwamm eine Antibiotikalösung zugeben kann (z.B. eine Gentamicinlösung) und damit ein formbares Knochenersatzmaterial mit Antibiotika erhält.

In einer weiteren bevorzugten Ausführungsform wird das formbare Knochenersatzmaterial (Putty) einer Gammabestrahlung von bevorzugt 25-40 kGray unterzogen, wodurch elastische Blöcke entstehen.

Gegenstand der Erfindung ist darüber hinaus ein formbares Knochenersatzmaterial (Putty), das ein als Knochenersatzmaterial nutzbares Granulat und ein, wie oben beschrieben hergestelltes, Biomaterial umfasst. Vorzugsweise wird das formbare Knochenersatzmaterial (Putty) nach den oben beschriebenen Verfahren hergestellt.

Die Erfindung wird in den folgenden Beispielen verdeutlicht und illustriert, jedoch in ihrem Rahmen dadurch nicht eingeschränkt. In dieser Anmeldung zitierte Publikationen sind durch die Referenz hierin vollständig eingeschlossen.

### Legenden

- Fig. 1: zeigt rasterelektronenmikroskopische Abbildungen des in Beispiel 1 hergestellten Schwamms aus Polyvinylpyrrolidon und SiO₂-Nanoteilchen in verschiedenen Vergrößerungen. A: Skala=200 µm, B: Skala= 40 µm, C: Skala= 9 µm.

### Beispiele

### Beispiel 1

Mit einem Kationenaustauscher (z.B. LEWATIT®) werden aus Natriumwasserglaslösung mit einem SiO₂ Anteil von 7% die Natriumionen entfernt. Es entsteht ein Sol mit einem pH Wert von ca. 2,4. Nach der Bestimmung der Feststoffkonzentration wird soviel Wasser hinzugefügt, dass ein Sol mit 6% SiO₂ Anteil entsteht. 200 g von diesem Sol werden mit 200 g einer zwölfprozentigen PVP Lösung homogen vermischt. Hierzu wird die Ultraschallhomogenisierung genutzt. Anschließend wird der pH Wert mit NaOH Lösung auf 7,4 eingestellt. Das Gel wird in Formen von z.B. 100 mm x 100 mm x 8 mm gegeben und gefriergetrocknet. Rasterelektronenmikroskopische Aufnahmen sind in Fig. 1 gezeigt.

### Beispiel 2

### Wasserlösliches Vlies zur Wundabdeckung

Mit einem Kationenaustauscher (z.B. LEWATIT®) werden aus Natriumwasserglaslösung mit einem SiO₂ Anteil von 7% die Natriumionen entfernt. Es entsteht ein Sol mit einem pH Wert von ca. 2,4. Nach der Bestimmung der Feststoffkonzentration wird soviel Wasser hinzugefügt, dass ein Sol mit 0,66% SiO₂ Anteil entsteht. 200 g von diesem Sol werden mit 200 g einer wässrigen 1.34 % igen PVP K 90 Lösung (PVP - Polyvinylpyrrolidon) homogen vermischt. Zum Mischen wird ein Rührer mit 1000 Umdrehungen pro Minute genutzt. Anschließend wird der pH Wert mit NaOH Lösung auf 7,4 +/- 0,5 eingestellt. Das Gel wird in Formen von z.B. 100 mm x 100 mm x 8 mm gegeben und gefriergetrocknet. Es entsteht ein Vlies zur Wundabdeckung.

### Beispiel 3

### Wasserunlösliches Vlies zur Wundabdeckung

Mit einem Kationenaustauscher (z.B. LEWATIT®) werden aus Natriumwasserglaslösung mit einem SiO₂ Anteil von 7% die Natriumionen entfernt. Es entsteht ein Sol mit einem pH Wert von ca. 2,4. Nach der Bestimmung der Feststoffkonzentration wird soviel Wasser hinzugefügt, dass ein Sol mit 0,66% SiO₂ Anteil entsteht. 200 g von diesem Sol werden mit 200 g einer wässrigen 1,34 % igen PVP K 90 Lösung (PVP - Polyvinylpyrrolidon) homogen vermischt. Zum Mischen wird ein Rührer mit 1000 Umdrehungen pro Minute genutzt. Anschließend wird der pH Wert mit NaOH Lösung auf 7,4 +/- 0,5 eingestellt. Das Gel wird in Formen von z.B. 100 mm x 100 mm x 8 mm gegeben und gefriergetrocknet. Anschließend wird das entstanden Vlies solange gestättigtem Wasserdampf ausgesetzt, bis es 10 % seines Gewichts Wasser aufgenommen hat (quellen). Die nachfolgende Gammabestrahlung (25-40 kGray) sorgt für eine Quervernetzung des PVP im Vlies. Es entsteht ein Vlies zur Wundabdeckung.

### Beispiel 4

### Gelartige Wundabdeckung

Mit einem Kationenaustauscher (z.B. LEWATIT®) werden aus Natriumwasserglaslösung mit einem SiO₂ Anteil von 7% die Natriumionen entfernt. Es entsteht ein Sol mit einem pH Wert von ca. 2,4. Nach der Bestimmung der Feststoffkonzentration wird soviel Wasser hinzugefügt, dass ein Sol mit 6% SiO₂ Anteil entsteht. 200 g von diesem Sol werden mit 200 g einer wässrigen 12 %igen PVP K 90 Lösung (PVP - Polyvinylpyrrolidon) homogen vermischt. Zum Mischen wird ein Rührer mit 1000 Umdrehungen pro Minute genutzt. Anschließend wird der pH Wert mit Na-OH Lösung auf 7,4 +/- 0,5 eingestellt. Das Gel wird in Formen von z.B. 100 mm x 100 mm x 8 mm gegeben und es wird einer Gammabestrahlung von 25-40 kGray unterzogen. Es entsteht eine gelartige feuchte Wundauflage.

### Beispiel 5

### Knochenersatzmaterial-Putty

Mit einem Kationenaustauscher (z.B. LEWATIT®) werden aus Natriumwasserglaslösung mit einem SiO₂ Anteil von 7% die Natriumionen entfernt. Es entsteht ein Sol mit einem pH Wert von ca. 2,4. Nach der Bestimmung der Feststoffkonzentration wird soviel Wasser hinzugefügt, dass ein Sol mit 6% SiO₂ Anteil entsteht. 50 g von diesem Sol werden mit 50 g einer wässrigen 12 %igen PVP K 90 Lösung (PVP - Polyvinylpyrrolidon) homogen vermischt. Zum Mischen wird ein Rührer mit 1000 Umdrehungen pro Minute genutzt. 62 g eines hoch porösen Knochenersatzmaterialgranulates (nach Patent EP 1 624 904 hergestellt) werden mit soviel Wasser versetzt, dass die inneren Poren sich füllen (in diesem Fall mit 44 g Wasser). Die Silica/PVP Mischung und das feuchte Granulat werden homogen vermischt. Anschließend wird der pH Wert mit Na-OH Lösung auf 7,4 +/- 0,5 eingestellt. Die Masse wird in typische Applikatoren für Knochenersatz abgefüllt und im Autoklaven sterilisiert.

### Beispiel 6

### Knochenersatzmaterial-Putty zum Anmischen mit handelüblichen Antibiotikalösungen

Mit einem Kationenaustauscher (z.B. LEWATIT®) werden aus Natriumwasserglaslösung mit einem SiO₂ Anteil von 7% die Natriumionen entfernt. Es entsteht ein Sol mit einem pH Wert von ca. 2,4. Nach der Bestimmung der Feststoffkonzentration wird soviel Wasser hinzugefügt, dass ein Sol mit 6% SiO₂ Anteil entsteht. 50 g von diesem Sol werden mit 50 g einer wässrigen 12 %igen PVP K 90 Lösung (PVP - Polyvinylpyrrolidon) homogen vermischt. Zum Mischen wird ein Rührer mit 1000 Umdrehungen pro Minute genutzt. 62 g eines hoch porösen Knochenersatzmaterialgranulates (hergestellt wie beschrieben im Europäischen Patent EP 1 624 904) werden mit soviel Wasser versetzt, dass die inneren Poren sich füllen (in diesem Fall mit 44 g Wasser). Die Silica/PVP Mischung und das feuchte Granulat werden homogen vermischt. Anschließend wird der pH Wert mit NaOH Lösung auf 7,4 +/- 0,5 eingestellt. Aus der Masse werden Zylinder geformt (10 mm Durchmesser, 30 mm lang). Diese Zylinder werden gefriergetrocknet. Es entstehen schwammartige trockene Körper, die in Applikatoren mit einem Innendurchmesser von 10 mm eingebracht werden. Es folgt eine dampfdichte Verpackung in handelüblichen Alu-Peelbeuteln und eine Gammasterilisation. Zur Anwendung wird in die schwammartigen Körper im Applikator mit einer Spritze soviel Antibiotikalösung gegeben, das keine Luft im Applikator ist (z.B.1,5 ml Gentamicin-ratiopharm® 40 SF Injektionslösung). Der schwammartige Zylinder quillt und ergibt eine knetbare Masse (Putty) zur Füllung von Knochendefekten.

### Beispiel 7

### Elastischer Knochenersatzmaterial-Formkörper

Mit einem Kationenaustauscher (z.B. LEWATIT®) werden aus Natriumwasserglaslösung mit einem SiO₂ Anteil von 7% die Natriumionen entfernt. Es entsteht ein Sol mit einem pH Wert von ca. 2,4. Nach der Bestimmung der Feststoffkonzentration wird soviel Wasser hinzugefügt, dass ein Sol mit 6% SiO₂ Anteil entsteht. 50 g von diesem Sol werden mit 50 g einer wässrigen 12 %igen PVP K 90 Lösung (PVP - Polyvinylpyrrolidon) homogen vermischt. Zum Mischen wird ein Rührer mit 1000 Umdrehungen pro Minute genutzt. 62 g eines hoch porösen Knochenersatzmaterialgranulates (hergestellt wie beschrieben im Europäischen Patent EP 1 624 904) werden mit soviel Wasser versetzt, dass die inneren Poren sich füllen (in diesem Fall mit 44 g Wasser). Die Silica/PVP Mischung und das feuchte Granulat werden homogen vermischt. Anschließend wird der pH Wert mit NaOH Lösung auf 7,4 +/- 0,5 eingestellt. Die Masse wird in Formen gegeben (Blisterverpackung 15 x 10 x 5 mm³. Es folgt eine dampfdichte Verpackung in handelüblichen Alu-Peelbeuteln und eine Gammasterilisation von bevorzugt 25-40 kGray. Es entstehen elastische Blöcke, die für die Knochenaugmentation verwendet werden.

## Patentansprüche

1. Verfahren zur Herstellung von gering vernetzten Kieselsäurestrukturen in einer Polymermatrix, **dadurch gekennzeichnet,**
a) **dass** man ein SiO₂ Sol in einem Lösungsmittel erzeugt, wobei die Solteilchen bevorzugt eine Größe von 0,5 nm bis 20 nm haben,
b) **dass** man eine Lösung eines Polymers in einem Lösungsmittel herstellt,
c) **dass** man die Lösung und das Sol homogen vermischt
wobei das Lösungsmittel Wasser oder Alkohol oder ein Gemisch aus Wasser und Alkohol ist, und wobei das Polymer Polyvinylpyrrolidon (PVP) ist, und wobei SiO₂ und Polymer in Schritt c) in einem Massenverhältnis (SiO₂/Polymer) von 0,5/99,5 bis 50/50 vorliegen.

2. Verfahren nach Anspruch 1, **dadurch gekennzeichnet, dass** man ein Sol erzeugt, und dass man eine weitere Kondensation im Sol verhindert, indem man eine Lösung eines löslichen Polymers hinzu gibt, wenn bestimmte Vernetzungen der Kieselsäure erreicht werden, wobei bevorzugt Kieselsäurestrukturen einer Größe von 0,5-20 nm in einer Polymermatrix hergestellt werden.

3. Verfahren nach einem der vorhergehenden Ansprüche, **dadurch gekennzeichnet, dass** die Kondensation der Kieselsäure in wässriger oder in alkoholischer Lösung so gesteuert wird, dass definierte Kieselsäurestrukturen entstehen, indem man eine Lösung eines löslichen Polymers hinzu gibt, wenn bestimmte Vernetzungen der Kieselsäure erreicht werden,
und wobei diese Polyederstrukturen bei den folgenden Verfahrensschritten wie z.B. dem Entfernen des Lösungsmittels erhalten bleiben, so dass gering vernetzte Kieselsäurestrukturen erzeugt werden, die nicht in einem kontinuierlichen Netzwerk eingebaut sind,
wobei bevorzugt Kieselsäurestrukturen einer Größe von 0,5-20 nm in einer Polymermatrix hergestellt werden, und/oder
wobei die Kieselsäurestrukturen bevorzugt Polyederstrukturen, mehr bevorzugt im wesentlichen SiO₂-Tetraeder sind, wobei Fünfer-, Sechs- und/oder Siebenerringe eine räumliche Struktur von ca. 0,5 nm Durchmesser bilden.

4. Verfahren nach einem der vorhergehenden Ansprüche, **dadurch gekennzeichnet, dass** das Polymer Polyvinylpyrrolidon K90 ist.

5. Verfahren nach einem der vorhergehenden Ansprüche, **dadurch gekennzeichnet,**
a) **dass** man das in Schritt c) hergestellte Gemisch trocknet; und/oder
b) **dass** man das in Schritt c) hergestellte Gemisch in eine Form gießt und gefriertrocknet; und/oder
c) **dass** Lösungsmittel aus dem in Schritt c) hergestellten Gemisch entfernt wird, bevorzugt soviel, dass die Viskosität im Bereich von 0,5 Pas bis 1 Pas liegt und/oder dass Fäden gesponnen werden, die bevorzugt in einem temperierten Gasstrom getrocknet werden, wobei bevorzugt aus den Fäden ein Vlies oder ein Gewebe hergestellt wird; und/oder
d) **dass** man den pH der in Schritt c) hergestellten Mischung von Sol und Polymerlösung auf ca. 6-8 einstellt.

6. Verfahren nach einem der vorhergehenden Ansprüche, **dadurch gekennzeichnet, dass** der Vernetzungsgrad des Polymers, bevorzugt des PVP, durch Gammabestrahlung erhöht wird.

7. Verfahren gemäß einem der vorhergehenden Ansprüche, **dadurch gekennzeichnet, dass** das Sol durch Hydrolyse von Tetraethylorthosilicat (TEOS) erzeugt wird, wobei bevorzugt ein r_{w} Wert (Molverhältnis von Wasser zu TEOS) von 4 genutzt wird, oder das Sol durch Ionenaustausch von Natriumwasserglaslösung erzeugt wird.

8. Biomaterial, erhältlich nach einem Verfahren nach einem der Ansprüche 1-7, welches aus gering vernetzten Kieselgelstrukturen aufgebaut ist, die bevorzugt eine Größe von 0,5 nm bis 20 nm aufweisen und in einer Polyvinylpyrrolidon-Polymermatrix verteilt sind.

9. Biomaterial nach Anspruch 8, **dadurch gekennzeichnet, dass** es aus SiO₂ Strukturen, bevorzugt Polyederstrukturen aufgebaut ist, die eine Größe von 0,5 nm bis 4 nm aufweisen, und die in einer Polymermatrix homogen verteilt sind

10. Biomaterial nach Anspruch 8 oder 9, **dadurch gekennzeichnet,**
a) **dass** es ein Hydrogel aufgebaut aus SiO₂ Polyederstrukturen, die eine Größe von 0,5 nm bis 4 nm aufweisen, und einem wasserlöslichen Polymer ist, wobei Polymer und SiO₂ Polyederstrukturen homogen verteilt sind; und/oder.
b) **dass** SiO₂ und Polymer im Massenverhältnis 0,5/99,5 (SiO₂/Polymer) bis 50/50 vorliegen; und/oder
c) **dass** das Polymer Polyvinylpyrrolidon, bevorzugt Polypvinylpyrrolidin K90, ist.

11. Biomaterial nach einem der Ansprüche 8 bis 10, **dadurch gekennzeichnet, dass** es als Fäden, die bevorzugt ein Vlies oder Gewebe bilden, als Folie oder als Schwamm vorliegt.

12. Biomaterial nach einem der Ansprüche 8 bis 11, **dadurch gekennzeichnet, dass** es zusammen mit als Knochenersatzmaterial nutzbaren Granulaten eine Masse zum Füllen von Knochendefekten (Putty) bildet.

13. Biomaterial nach einem der Ansprüche 8-12 zur Verwendung
a) als Medizinprodukt, das eine stützende oder abschirmende Funktion hat und bevorzugt gleichzeitig durch die Degradation als Lieferant für das die Geweberegeneration unterstützende Siliziumdioxid dienen kann; und/oder
b) für die Behandlung von Wunden oder Narben oder für kosmetische Anwendungen, insbesondere als Salbe oder Creme.

14. Medizinprodukt oder ein Nahrungsergänzungsmittel, umfassend das Biomaterial nach einem der Ansprüche 8-13.

15. Verfahren zur Herstellung eines formbaren Knochenersatzmaterials (Putty), bei dem man
a) ein als Knochenersatzmaterial nutzbares Granulat, welches bevorzugt poröses Material, ein Calciumphosphat und/oder Hydroxylapatitkeramik umfasst oder bovinen Ursprungs ist, mit einer wässrigen Lösung anfeuchtet, und
b) das als Knochenersatzmaterial nutzbare Granulat mit einem Biomaterial nach einem der Ansprüche 8-13 vermischt.

16. Verfahren nach Anspruch 15, bei dem das formbare Knochenersatzmaterial (Putty) gefriergetrocknet wird und/oder einer Gammabestrahlung von bevorzugt 25-40 kGray unterzogen wird.

17. Ein formbares Knochenersatzmaterial (Putty), das ein als Knochenersatzmaterial nutzbares Granulat und ein Biomaterial nach einem der Ansprüche 8-13 umfasst.

## Claims

1. Method of preparing silicic acid structures with a low degree of cross-linking in a polymer matrix, comprising
a) producing an SiO₂ sol in a solvent, wherein the sol particles preferably have a size of 0.5 nm to 20 nm,
b) producing a solution of a polymer in a solvent,
c) mixing the solution and the sol homogeneously,
wherein the solvent is water or alcohol or a mixture of water and alcohol, and wherein the polymer is polyvinylpyrrolidone (PVP), and wherein SiO₂ and polymer in step c) are present in a mass ratio (SiO₂/polymer) of 0.5/99.5 to 50/50.

2. Method according to claim 1, comprising producing a sol, and further preventing condensation in the sol by adding a solution of a soluble polymer once specific degrees of cross-linking of the silicic acid have been reached, wherein preferably silicic acid structures having a size of 0.5-20 nm in a polymer matrix are produced.

3. Method according to any of the preceding claims, comprising controlling the condensation of the silicic acid in aqueous or alcoholic solution such that defined silicic acid structures are produced by adding a solution of a soluble polymer when a defined cross-linking of the silicic acid is achieved,
and wherein the polyhedron structures are retained in the following process steps, such as removal of the solvent, so that silicic acid structures with a low degree of cross-linking are produced which are not incorporated in a continuous network,
wherein preferably silicic acid structures with a size of 0.5-20 nm are produced in a polymer matrix, and/or
wherein the silicic acid structures are preferably polyhedron structures, more preferably essentially SiO₂ tetrahedrons, wherein rings of five, six and/or seven form a spatial structure of about 0.5 nm diameter.

4. Method according to any of the preceding claims, wherein the polymer is polyvinylpyrrolidone K90.

5. Method according to one of the preceding claims, comprising
a) drying the mixture produced in step c); and/or
b) pouring the mixture produced in step c) into a mold and freeze-drying it; and/or
c) removing solvent from the mixture produced in step c), preferably to such an extent that the viscosity is in the range from 0.5 Pas to 1 Pas, and/or comprising spinning threads that are dried in a tempered gas stream, wherein a non-woven fabric or a woven fabric is produced from the threads; and/or
d) adjusting the pH of the mixture of sol and polymer solution prepared in step c) to about 6-8.

6. Method according to any of the preceding claims, wherein the degree of cross-linking of the polymer, preferably PVP, is increased by gamma irradiation.

7. Method according to one of the preceding claims, wherein the sol is produced by hydrolysis of tetraethylorthosilicate (TEOS), preferably using an r_{w} value (molar ratio of water to TEOS) of 4, or the sol is produced by ion exchange of sodium waterglass solution.

8. Biomaterial, obtainable by a process according to one of claims 1-7, which is composed of silicic acid structures with a low degree of cross-linking which preferably have a size of 0.5 nm to 20 nm and are distributed in a polyvinylpyrrolidone polymer matrix.

9. Biomaterial according to claim 8, which is composed of SiO₂ structures, preferably polyhedron structures which have a size of 0.5 nm to 4 nm and are homogeneously distributed in a polymer matrix.

10. Biomaterial according to claim 8 or 9, wherein
a) it is a hydrogel composed of SiO₂ polyhedron structures having a size of 0.5 nm to 4 nm and a water-soluble polymer, wherein the polymer and the SiO₂ polyhedron structures are homogeneously distributed; and/or
b) SiO₂ and polymer are present in a mass ratio of 0.5/99.5 (SiO₂/polymer) to 50/50; and/or
c) wherein the polymer is polyvinylpyrrolidone, preferably polyvinylpyrrolidone K90.

11. Biomaterial according to one of claims 8 to 10, which is present as threads, which preferably form a fleece or fabric, as a film or as a sponge.

12. Biomaterial according to one of claims 8 to 11, which in combination with granules, that are usable as bone replacement material, forms a mass for filling bone defects (putty).

13. Biomaterial according to any of claims 8-12 for use
a) as a medical device, which has a supporting or shielding function and, due to degradation, can preferably also serve as a supplier of the silicon dioxide that supports tissue regeneration; and/or
b) for the treatment of wounds or scars or for cosmetic applications, in particular as an ointment or cream.

14. A medical device or a food supplement comprising the biomaterial according to any of claims 8-13.

15. Process for the preparation of a mouldable bone replacement material (putty), comprising
a) moistening a granulate usable as a bone replacement material, which preferably comprises porous material, a calcium phosphate and/or hydroxyapatite ceramic or is of bovine origin, with an aqueous solution, and
b) mixing the granulate, which is usable as bone replacement material, with a biomaterial according to one of the claims 8-13.

16. Method according to claim 15, wherein the mouldable bone replacement material (putty) is freeze-dried and/or subjected to a gamma irradiation of preferably 25-40 kGray.

17. Mouldable bone replacement material (putty) comprising a granulate useful as a bone replacement material and a biomaterial according to any of claims 8-13.

## Revendications

1. Procédé visant à former des structures d'acide silicique peu réticulées dans une matrice de polymère, **caractérisé**
a) **en ce qu'**on produit un sol de silice SiO₂ dans un solvant, sol dont les particules ont de préférence une taille de 0,5 à 20 nm,
b) **en ce qu'**on prépare une solution d'un polymère dans un solvant,
c) et **en ce qu'**on mélange la solution et le sol jusqu'à homogénéité, dans lequel procédé
- le solvant est de l'eau, un alcool ou un mélange d'eau et d'alcool,
- le polymère est de la poly(vinyl-pyrrolidone) ou PVP,
- et dans l'étape (c), la silice et le polymère se trouvent présents en un rapport massique SiO₂/polymère valant de 0,5/99,5 à 50/50.

2. Procédé conforme à la revendication 1, **caractérisé en ce que** l'on produit un sol et l'on empêche toute condensation supplémentaire au sein du sol en y ajoutant une solution d'un polymère soluble quand est atteint un certain degré de réticulation de l'acide silicique, grâce à quoi il se forme de préférence des structures d'acide silicique de 0,5 à 20 nm de taille dans une matrice de polymère.

3. Procédé conforme à l'une des revendications précédentes, **caractérisé en ce que** l'on maîtrise la condensation de l'acide silicique dans la solution aqueuse ou alcoolique de manière qu'il se forme des structures d'acide silicique définies, en y ajoutant une solution d'un polymère soluble quand est atteint un certain degré de réticulation de l'acide silicique, étant entendu
- que ces structures polyédriques se conservent en l'état lors des étapes ultérieures de procédé, comme par exemple l'élimination du solvant, de sorte qu'il se forme des structures d'acide silicique peu réticulées qui ne sont pas intégrées en un réseau continu,
- qu'il se forme de préférence des structures d'acide silicique de 0,5 à 20 nm de taille dans une matrice de polymère,
- et/ou que les structures d'acide silicique sont de préférence des structures polyédriques, et mieux encore, essentiellement des tétraèdres SiO₂, dont des anneaux de cinq, six et/ou sept forment une structure volumique d'environ 0,5 nm de diamètre.

4. Procédé conforme à l'une des revendications précédentes, **caractérisé en ce que** le polymère est de la poly(vinyl-pyrrolidone) K90.

5. Procédé conforme à l'une des revendications précédentes, **caractérisé**
a) **en ce qu'**on fait sécher le mélange préparé dans l'étape (c),
b) et/ou en ce qu'on verse dans un moule le mélange préparé dans l'étape (c) et on le lyophilise,
c) et/ou en ce qu'on chasse le solvant du mélange préparé dans l'étape (c), de préférence à tel point que la viscosité du mélange se situe dans l'intervalle allant de 0,5 à 1 Pa.s, et/ou qu'on en fait des fils qui sont de préférence séchés dans un courant de gaz tempéré, fils à partir desquels, de préférence, on fabrique un voile ou un tissu,
d) et/ou en ce qu'on ajuste à une valeur d'à peu près 6 à 8 le pH du mélange de sol et de solution de polymère préparé dans l'étape (c).

6. Procédé conforme à l'une des revendications précédentes, **caractérisé en ce que** l'on élève le degré de réticulation du polymère, de préférence de la PVP, par irradiation avec des rayons gamma.

7. Procédé conforme à l'une des revendications précédentes, **caractérisé en ce que** l'on produit le sol par hydrolyse d'ortho-silicate de tétraéthyle (TEOS), en opérant de préférence avec une valeur de 4 du rapport molaire r_{w} de l'eau au TEOS, ou l'on produit le sol par échange d'ions à partir d'une solution de verre soluble au sodium.

8. Biomatériau, accessible par un procédé conforme à l'une des revendications 1 à 7, qui est constitué de structures de gel de silice peu réticulées qui, de préférence, présentent une taille de 0,5 à 20 nm et sont réparties dans une matrice de polymère poly(vinyl-pyrrolidone).

9. Biomatériau conforme à la revendication 8, **caractérisé en ce qu'**il est constitué de structures de SiO₂, de préférence des structures polyédriques, qui présentent une taille de 0,5 à 4 nm et sont réparties de manière homogène dans une matrice de polymère.

10. Biomatériau conforme à la revendication 8 ou 9, **caractérisé**
a) **en ce que** c'est un hydrogel constitué de structures polyédriques de SiO₂ présentant une taille de 0,5 à 4 nm, et d'un polymère hydrosoluble, dans lequel hydrogel le polymère et les structures polyédriques de SiO₂ sont répartis de manière homogène,
b) et/ou en ce que la silice SiO₂ et le polymère sont présents en un rapport massique SiO₂/polymère valant de 0,5/99,5 à 50/50,
c) et/ou en ce que le polymère est de la poly(vinyl-pyrrolidone), et de préférence de la poly(vinyl-pyrrolidone) K90.

11. Biomatériau conforme à l'une des revendication 8 à 10, **caractérisé en ce qu'**il se présente sous l'aspect de fils, de préférence formant un voile ou un tissu, ou sous l'aspect de feuille ou d'éponge.

12. Biomatériau conforme à l'une des revendication 8 à 11, **caractérisé en ce qu'**il forme, conjointement avec un granulat utilisable comme matériau substitut osseux, une masse conçue pour combler des défauts osseux (mastic).

13. Biomatériau conforme à l'une des revendications 8 à 12, pour utilisation
a) en tant que produit médical qui tient une fonction de soutien ou de protection et qui, de préférence, peut en même temps servir, en se dégradant, de pourvoyeur de dioxyde de silicium aidant à la régénération de tissus,
b) et/ou dans le traitement de plaies ou de cicatrices ou dans des applications cosmétiques, en particulier sous forme de pommade ou de crème.

14. Produit médical ou complément nutritionnel, qui comprend un biomatériau conforme à l'une des revendications 8 à 13.

15. Procédé de préparation d'un matériau substitut osseux façonnable (mastic), dans lequel procédé
a) on humecte avec une solution aqueuse un granulat utilisable comme matériau substitut osseux, qui comprend de préférence un matériau poreux, un phosphate de calcium et/ou une céramique d'hydroxyapatite, ou qui est d'origine bovine,
b) et l'on mélange ce granulat utilisable comme matériau substitut osseux avec un biomatériau conforme à l'une des revendications 8 à 13.

16. Procédé conforme à la revendication 15, dans lequel le matériau substitut osseux façonnable (mastic) est lyophilisé et/ou soumis à une irradiation avec des rayons gamma, de préférence à une dose de 25 à 40 kilograys.

17. Matériau substitut osseux façonnable (mastic), comprenant un granulat utilisable comme matériau substitut osseux et un biomatériau conforme à l'une des revendications 8 à 13.
